# EUROPEAN PATENT APPLICATION

(11) **EP 2 133 104 A1**
(43) Date of publication of application: **16.12.2009**
(21) Application number: 07003868.2
(22) Date of filing: 26.02.2007
(51) Int. Cl.: A61L 9/12, A47K 10/32, B65H 75/18

(54) **Core for Rolls**

(30) Priority: 02.03.2006 IT MO20060075
(71) Applicant: Cartiera Lucchese S.p.A., 55016 Porcari LU (IT)
(72) Inventor: Pasquini, Sandro, 55100 Lucca (IT)
(74) Representative: Brogi, Graziano

(57) **Abstract**

Core (1) for rolls (2), especially toilet paper rolls, absorbent paper rolls, rolls of film materials and rolls of metal materials, whereby the inner surface (4) of the core (1) is provided with deodorant and/or perfume substances to be released into the environment. These substances can be provided in containment means such as a strip (6) or absorbent elements (7), coupled to said inner surface (4).

## Description

The invention relates to a core for rolls, suitable for releasing substances into the environment, particularly deodorant and/or perfume substances.

The rolls of materials which can be used for domestic and industrial uses, such as toilet paper rolls, absorbent paper rolls, rolls of film materials, are normally shaped by wrapping the materials around a central core which is made up of a segment of tube made of different materials, depending on the type of material wrapped and the intended use of these materials: this tube segment is normally made of cardboard or plastic.

The rolls of materials wrapped around these cores for their use can be placed in environments where unpleasant odours with a tendency to stagnate are generated, e.g., the environments where there are toilet facilities or where food is cooked, especially if the latter is fried, or also in restaurants, cafés and bars, medical surgeries, and public toilets.

To eliminate these unpleasant odours, aspirators with different powers are used which suck in air full of these unpleasant odours and blow it outside these environments and, in addition to the action of these aspirators, deodorant and perfume substances are spread into the environments.

To spread these substances cans can be used that contain and atomize them or suitable containers can be used which internally preserve the substances in the liquid or gelatinous state and which have an openable lid, through which the substances spread slowly and over a substantially extended length of time.

Another way of spreading the substances is to prepare supports made of absorbent materials which are soaked with these substances and to place these supports in the environments where these substances are to be released.

This state of the art does have some drawbacks.

A first drawback is that to get a deodorizing and perfuming action of environments, it is necessary to buy specific ready-made devices to obtain such action.

Another drawback is that to make deodorant devices it is necessary to use containers and supports of various kind made out of various materials to contain the substances and to release them into the environment: these containers weigh considerably on the cost of the deodorant devices and therefore make expensive to have the deodorizing and perfuming action.

A further drawback is that when they are empty, the deodorant devices must be disposed of in containers specially intended for selective waste collection of the materials out of which they have been made and, for this reason, they must be separately collected in advance from other objects for normal waste disposal.

Another drawback is that these deodorant and perfume devices require a special area to place them in an environment.

One object of the invention is to improve the state of the art.

Another object of the invention is to provide for a core for rolls allowing to obtain a spreading action of substances into the environment, in this specific case of deodorant and/or perfume substances without the need to buy special spreading devices.

A further object of the invention is to provide for a core for rolls which, when the rolls are used up, can be disposed of without particular precautions having been made with environment-friendly materials.

According to one aspect of the invention a core for rolls is provided characterized in that it comprises substances that can be released into the environment.

According to another aspect of the invention a method is provided for shaping cores for rolls characterized in that it comprises endowing these cores with substances that can be released into the environment.

According to a further aspect of the invention a roll of wrapped materials around a support core is provided characterized in that said core comprises substances that can be released into the environment.

Therefore, the core for rolls allows for releasing substances into the environment, specifically deodorant and/or perfume substances, without having to buy additional ready-made devices to release these substances.

Further characteristics and advantages of this invention will appear even more evident from the detailed description of a preferred, but not exclusive, embodiment of a core for rolls, suitable for releasing substances into the environment, particularly deodorant and/or perfume substances, illustrated indicatively by way of non limiting example, in the attached drawings wherein:
Figure 1 is a perspective view of a core for rolls extracted from a roll of toilet paper;
Figure 2 is a schematic perspective view of a phase of shaping the core of Figure 1 in a first possible embodiment;
Figure 3 is a cross section view of the core of Figure 2, taken according to a sketch plan III-III.

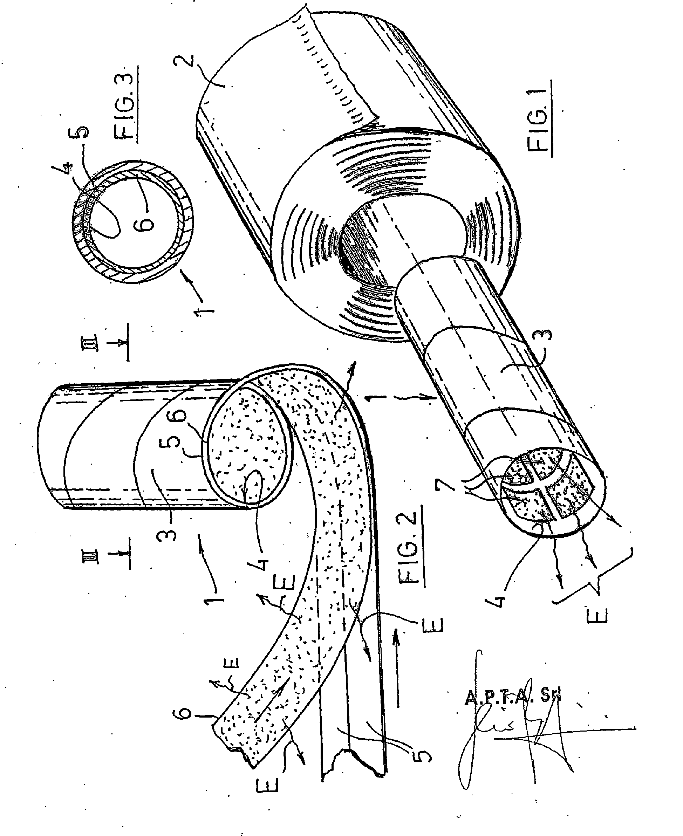

With reference to Figure 1, by reference number 1 is designated a core for rolls 2 which comprises a body 3 shaped like a tubular segment and internally run through by an axial cavity 4.

The core 1, in the version illustrated in the Figures, is made by wrapping, as indicated in detail in Figure 2, two cardboard strips 5 around a shaping spindle, not shown in the Figures, along a helical direction and, to make it able to release substances into the environment, normally deodorant and/or perfume substances, the cardboard strip 5 is coupled with an additional strip 6 of absorbent material that contains the substances to release.

In this version of the core 1, the cardboard strips 5 and the additional strip 6 of absorbent material are coupled together, e.g. by means of a gluing material interposed between them, so that, when the core 1 is shaped, the additional strip 6 is inside the axial cavity 4 and, through this, can release into the environment the substance or the substances that has/have been previously introduced into such additional strip 6.

To keep such substances integral until the instant when the role 2 is used, the possibility is also envisaged to endow the additional strip 6 with a laminated covering which insulates it from the environment and which is of the peelable type, so that, when the role 2 is used, the laminated covering is removed and the additional strip 6 can activate the releasing action, as indicated by the arrows "E" in Figures 1 and 2.

In a second possible embodiment of the core 1, shown in Figure 1, instead of the additional strip 6, portions 7 of absorbent elements are arranged inside the axial cavity 4. These portions can have shape, dimensions, arrangement and density at will and can be endowed with a contact adhesive surface with the wall of the axial cavity 4: for this reason, the portions 7 can be applied before or after shaping the core 1.

According to a further possible embodiment of the latter, the core 1 itself is made of absorbent material soaked with the substances to release; in this case, the substances are introduced directly in the strip 5, which is made of absorbent material and sufficiently stiff, before it shapes the core 1, following wrapping around the respective shaping spindle.

When the core 1 is shaped, a material, e.g. paper in the form of strip, is wrapped around this in the known way, until shaping a roll 2 which, therefore, when subsequently used, releases into the environment where it is placed the substance or the substances to release with which the core 1 has been endowed.

The method for shaping cores 1 endowed with substances to release provides for, according to a first embodiment, the coupling by means of gluing, of a pair of strips 5 of a material such as cardboard, fed by means of a first feeder that is not shown, and an additional strip 6 of absorbent material, previously soaked with the substances to release and fed by a second feeder.

Both strips 5 and additional strip 6 are then wrapped together around a shaping spindle thus shaping the tubular body 3 which is then cut to size.

According to a further embodiment, the core 1 is first shaped by wrapping the cardboard strips 5 around a shaping spindle, then the tubular body 3 is cut to size and the portions 7 are subsequently applied having a surface endowed with adhesive material.

According to a further embodiment of the core 1, two strips 5 of absorbent material are used which are soaked with substances to release into the environment and which, subsequently, are wrapped in the known way around a shaping spindle to shape the tubular body 3.

When the core 1 is completed, it is placed onto an automatic machine suitable for wrapping materials on this until shaping rolls 2 of these materials.

## Claims

1. Core (1) for rolls (2) **characterized in that** it comprises substances which can be released into the environment.

2. Core (1) for rolls (2) according to claim 1 wherein said substances comprise deodorant and/or perfume substances.

3. Core (1) for rolls (2) according to claim 1 or 2 wherein said core comprises a body (3) to which said deodorant and/or perfume substances are applied.

4. Core (1) for rolls (2) according to any of the claims from 1 to 3 wherein said body comprises a cylindrical segment (3) which defines an outer surface and a hollow inner surface (4).

5. Core (1) for rolls (2) according to any of the claims from 1 to 3 wherein said deodorant and/or perfume substances are contained into containment means (6; 7) applicable to said body (3).

6. Core (1) for rolls (2) according to claim 5 wherein said containment means comprise a strip (6) soaked with said deodorant and/or perfume substances and which can be coupled with said body (3) with coupling means.

7. Core (1) for rolls (2) according to claim 5 wherein said containment means comprise absorbent elements (7) soaked with said deodorant and/or perfume substances and which can be coupled with said body (3) with coupling means.

8. Core (1) for rolls (2) according to claim 6 or 7 wherein said coupling means comprise adhesive means.

9. Core (1) according to claim 7 wherein said absorbent elements (7) are endowed with removable protection means before spreading said deodorant and/or perfume substances into the environment.

10. Core (1) for rolls (2) according to claim 5 wherein said containment means (7) are applicable on said inner surface (4).

11. Method for shaping cores (1) for rolls (2)
**characterized in that** it comprises endowing said cores (1) with substances which can be released into the environment.

12. Method according to claim 11 wherein said substances comprise deodorant and/or perfume substances.

13. Method according to claim 11 or 12 wherein said endowing comprises incorporating said deodorant and/or perfume substances in said cores (1).

14. Method according to claim 11 or 12 wherein said endowing comprises applying said deodorant and/or perfume substances to said cores (1).

15. Method according to claim 14 wherein said applying comprises: placing said deodorant and/or perfume substances on support means (6; 7); fastening said support means (6; 7) to said cores (1).

16. Method according to claim 15 wherein said fastening occurs during a shaping of said cores (1).

17. Method according to claim 15 wherein said fastening occurs after a shaping of said cores (1).

18. Roll (2) of materials wrapped around a support core (1), **characterized in that** said core (1) comprises substances which can be released into the environment.

19. Roll according to claim 18 wherein said substances which can be released comprise deodorant and/or perfume substances.

20. Roll (2) according to any of the claims from 1 to 18 wherein said roll (2) is shaped with paper material wrapped around said core (1).

21. Roll (2) according to any of the claims from 1 to 18 wherein said roll (2) is shaped with plastic film material wrapped around said core (1).

22. Roll (2) according to any of the claims from 1 to 18 wherein said roll (2) is shaped with thin flexible metal material in the form of a strip wrapped around said core (1).
